# EUROPEAN PATENT APPLICATION

(11) **EP 3 336 849 A1**
(43) Date of publication of application: **20.06.2018**
(21) Application number: 17183355.1
(22) Date of filing: 26.07.2017
(51) Int. Cl.: G16H 40/60, G16H 40/63, G16H 40/67

(54) **METHOD FOR USING A MEDICAL INSTRUMENT, SYSTEM COMPRISING A MEDICAL INSTRUMENT AND A REMOTE SIDE, COMPUTER PROGRAM PRODUCT AND COMPUTER-READABLE MEDIUM**

(71) Applicant: Siemens Healthcare GmbH, 91052 Erlangen (DE)
(72) Inventor: Aschoff, Katharina, 91052 Erlangen (DE); Faust, Dieter, 91056 Erlangen (DE); Kröll, Maria, 91052 Erlangen (DE); Nufer, Stephan, 91056 Erlangen (DE)

(57) **Abstract**

Method for using a medical instrument, System comprising a medical instrument and a remote side, Computer program product and computer-readable medium

The present invention suggest a method for using a medical instrument (10), in particular a medical imaging device, wherein the medical instrument (10) is selectively operable in a first mode giving access to the medical instrument (10) and in a second mode giving limited access, in particular no access, to the medical instrument (10), comprising the steps:
- requesting access to the medical instrument (10);
- transferring the medical instrument (10) into the first mode or into a second mode in response to the request by using a human-machine-interface (11) of the medical instrument (10) and
- transferring the medical instrument (10) into the first mode or into a second mode by using a human-machine interface (21) at a remote side (20), when the human-machine-interface (11) of the medical instrument (10) is inactive for a defined period of time after requesting the access.

## Description

The present invention describes a method for using a medical instrument, a system comprising a medical instrument and a remote side, a computer program product and a computer readable medium.

Medical imaging instruments, such as a Magnetic Resonance Tomography (MRT)-scanner, a X-ray-scanner, a Computer Tomography (CT)-scanner or similar devices, are well known in the state of the art. Typically, a first operator being present at the medical instrument operates the medical imaging device by using a human-machine-interface of the medical imaging device. For example, the first operator can start and finish a recording of a medial image, can configure the settings of the medical imaging device and/or can protocol the recorded images. For giving a second operator the chance to operate the medical imaging device from a remote side, the first operator has to choose between refusing an access of the second operator to the medical imaging device or selecting an extent of the access intended for the second operator. As a result, the first operator needs to be available at the human-machine-interface of the medical imaging device, when the second operator wants to access the medical instrument.

On a very general level, remote service aspects in connection with medical imaging devices are also disclosed in DE 102012203975 A1, DE 102012202362 A1, DE102012205296 A1 and DE 102014220808 B4 for example.

It is therefore an object of the present invention to provide a method for getting access to a medical instrument from a remote side in an unattended state of the medical instrument.

This object is achieved by the method according to claim 1 for using a medical instrument, by the system according to claim 13, the computer program product according to claim 14 and by the computer readable computer medium according to claim 15.

According to a first aspect of the present invention a method for using a medical instrument, in particular a medical imaging device, is provided, wherein the medical instrument is selectively operable in a first mode giving access to the medical instrument and in a second mode giving limited access, in particular refuse access, to the medical instrument, comprising the steps:
- requesting access to the medical instrument;
- transferring the medical instrument into the first mode or into the second mode in response to the request by using a human-machine-interface of the medical instrument, i.e. in a attended mode, and
- transferring the medical instrument into the first mode by using a human-machine interface at a remote side, when the human-machine-interface of the medical instrument is inactive for a defined period of time, in particular after requesting the access, i.e. in a unattended mode.

In contrast to the state of the art, the medical instrument is transferred into the first mode by the remote side, when the human-machine-interface is inactive, in particular for the defined period of time. As a consequence a second operator can access the medical instrument via the human-machine-interface at the remote side, even when a first operator does not interact with the human-machine interface, for example because the human-machine-interface of the medical instrument is unattended. Preferably, the medical instrument is a medical imaging device, such as a Magnetic Resonance Tomography (MRT)-scanner, a X-ray-scanner, a Computer Tomography (CT)-scanner or a similar device, and for instance operating the medical instrument means configuring a setting of the medical instrument, starting or finishing a recording of a medical imaging, protocolling the medical imaging and administering the medical imagines in a memory device, in particular by using the human-machine-interface of the medical instrument or the human-machine-interface of the remote side. Such a human-machine-interface might comprise a touchscreen, a tablet, a smartphone, a keyboard or a console. Preferably, it is provided that the remote side has full access in the first mode and as a consequence the second operator can operate the medical instrument, wherein the remote side has only access to a current image on a display of the medical instrument or the access is refused in the second mode. Preferably, in the first mode and/or the second mode specified functions, such as recording a medical imaging or moving a table of the medical instrument, are prevented or blocked. It is also conceivable that there are several second modes having each different extent of access to the medical instrument and the medical instrument is transferred to one of the several second modes. In particular, the extent of access in the first mode is bigger than the extent of access in the second mode or one of the several second modes.

The term "remote side" preferably comprises a computer or workstation being located spatially apart from the medical instrument, for example being located in another room, another floor and/or another building. Preferably, the medical instrument and the remote side exchange data and/or commands via a communication channel, such as a wireless or a wired connection. Furthermore, the medical instrument comprises a control unit for operating the medical instrument, wherein the control unit has a processor configuring a setting of the medical instrument, starting and finishing the recording of a medical imaging, protocolling the medical imaging and administering the medical imagines in a memory device.

The term "inactive" preferably describes a state, in which the human-interface-machine is not used and/or no selection between the first mode and the second mode has been chosen by using the human-machine interface during the defined period of time. Especially the period of time is triggered or started by the request. For example, the period of time lasts between 0.5 minutes and 10 minutes, preferred 0.7 and 5 minutes and most preferred about 1 min. In particular, such a request is initiated by the human-machine-interface at the remote side. Preferably, the first operator is informed about the request on a screen of the medical instrument immediately.

Particularly advantageous embodiments and features of the invention are given by the dependent claims as revealed in the following description. Features of different claim categories may be combined as appropriate to give further embodiments not described herein.

According to a preferred embodiment, it is provided that the method further comprises the step of transferring the medical instrument into the first mode, when the human-machine-interface is inactive for a defined period of time after requesting the access and the medical instrument is in a non-use state. Thus, it is avoided that the remote side configures accidentally the settings of the medical instrument during operation, in particular during recording or configuring the settings for a patient in the unattended state. For example, the medical instrument is in a use-state, when a patient is logged in, and is in a non-use state, when no patient is logged in. Preferably, the remote side is informed whether the medical instrument is in the use-state or the non-use state at the point of time of the request.

In a further embodiment of the present invention, it is provided that the medical instrument is transferred into the first mode or into the second mode automatically, when the human-machine-interface is inactive for a defined period of time before the request. Thus, the second operator can directly access the medical instrument, without waiting for expiration of the set period of time after requesting the time. In other words: The period of time in which the human-machine-interface is inactive already expired before the request. It is also thinkable that the defined period of time that has to expire before having access is reduced when the medical instrument was inactive before the request.

Preferably, an information about a remaining time in the first mode or the second mode is provided at the remote side. Thus, the second operator is informed about the time left for finishing his project or and as consequence he can organize his work effectively. In particular, a countdown is provided informing the second operator. It is also thinkable, that an alarm for example an acoustical or visual alarm is realized at defined points of time during the countdown.

According to another embodiment of the present invention it is provided that the first mode and/or the second mode are terminated by the human-machine-interface at the medical instrument or by the human-machine-interface at the remote side. In particular, terminating the first mode and/or second mode by the human-machine-interface of the medical instrument allows observing the actions of the second operator and stopping the access of the second operator for safety reason. In the case the first mode and/or the second mode is terminated by the human-machine-interface at the remote side, it is preferably provided that the medical instrument, in particular the first operator at the medical instrument, is informed about the termination and the first operator can resume its work.

Preferably, it is provided that transferring the medical instrument into the first mode or into the second mode, in particular into one of several second modes, by using a human-machine interface at a remote side is selected by a management system. Thus, it is possible to select the first mode or the second mode depending on the respective type of second operator. Therefore, the management system is configured such that the first mode, the second mode and/or one of the several second modes are assigned to the respective second operator. Thus, it is possible that the management system determines the first mode, the second mode and/or the individual second mode to the respective second operator individually. It is also possible that the management system is configured such that a second operator can identify himself by a password and subsequently the management system determines the corresponding first mode, the second mode and/or the one of the several second modes based on the password.

In another embodiment, it is provided that the management system is incorporated into the medical instrument or into a server. By incorporating the management system into the medical instrument, the first operator or an administrator of the medical devices can determinate the extent of access for the individual second operator, without the need of an additional component including the management system. Alternatively, it is also possible to incorporate the management system into a server, such as a network or a cloud. As a consequence, it is possible to establish a central assignment between the amount of access and the individual second operator, for example for a plurality of medical instruments.

Preferably, it is provided that a bidirectional communication between the medical instrument and the remote side is established in the first mode and a unidirectional communication is established in the second mode. Thus, the second operator cannot modify the medical instrument in the second mode. In particular, data corresponding to the content of the current screen of the medical instrument are transferred to the remote side in the second mode.

According to another embodiment, it is provided that an information about the present usage of the medical instrument is provided at the remote side. Thus, it is possible to inform the second operator about the status of the current recording process and/or the planned recordings, for example, and the second operator can approximate the time until the medical instrument is in a non-use state.

In a further embodiment, it is provided that in the first mode and/or the second mode administrative tasks are performed. Administrative tasks are for example a maintenance procedure or a protocol maintenance.

Another aspect of the present invention is a system comprising a medical instrument and a remote side, wherein the system is configured for
- requesting access;
- transferring the medical instrument into the first mode or into the second mode in response to the request by using a human-machine-interface at the medical instrument or
- transferring the medical instrument into the first mode or into the second mode by using a human-machine interface at a remote side, when the human-machine-interface is inactive, in particular for a defined period of time after requesting the access.

A further aspect of the present invention is a computer program product for carrying out the steps of the method according to the present invention, when the computer program product is loaded into a memory of a programmable device.

A further aspect of the present invention is a computer-readable medium on which is stored a program elements that can be read and executed by a computer unit in order to perform steps of the method according to the present invention when the program elements are executed by the computer unit.

In the drawings:

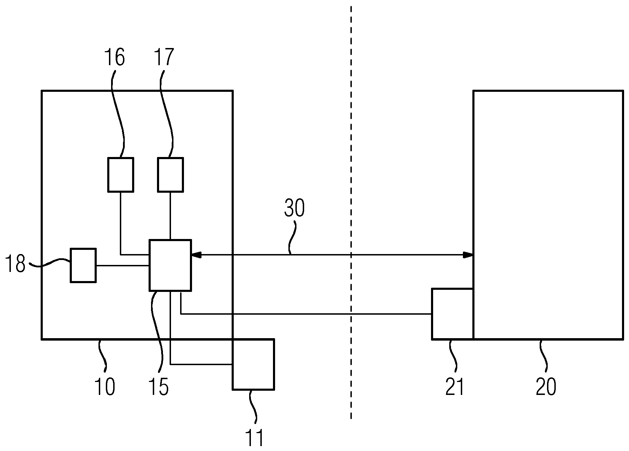

- Fig. 1: shows a block diagram illustrating schematically a method for configuring a medical instrument according to a preferred embodiment of the present invention.

In figure 1 a block diagram illustrating schematically a method for using a medical instrument 10 according to a preferred embodiment of the present invention is shown. For example, the medical instrument 10 is a medical imaging device such as a Magnetic Resonance Tomography (MRT) scanner, a X-ray-scanner, a Computer Tomography (CT)-scanner or a similar device. In particular, such a medical instrument 10 comprises a control unit 15 for configuring the setting of the medical instrument and operating the medical instrument. Preferably, for operating the medical instrument the control unit 15 has a processor being configured such that settings 16 of the medical instrument can be configured, a clinical task 18 of the medical instrument can be started, observed and finished, data can be stored in a memory device 17 and/or data can be manipulated. The term "data" preferably comprises an image data set and/or patient related information such as a name or an identification of the patient. Furthermore, the medical instrument comprises a human-machine-interface 11 being connected to the control unit 15, communicatively. Such a human-machine-interface 11 might comprise a touchscreen, a tablet, a smartphone, a keyboard or a console. Via the human-machine-interface a first operator of the medical instrument has access to the control unit for operating the medical instrument.

Furthermore, the system shown in figure 1 comprises a remote side 20. For a communication, i. e. an exchange of data sets and/or or commands, between the medical instrument 10 and the remote side 20 a communication channel 30 is provided. Such a communication channel 30 can be realized by a wireless connection or a wired connection. In particular, the remote side 20 is located spatially apart from the medical instrument 10, for example in another room or floor. For operating the medical instrument 10 by a second operator at the remote side 20, the remote side 20 comprises also a human-machine-interface 21.

A prerequisite for operating the medical instrument 1 by the remote side 20 is that access to the medical instrument 10, in particular to the control unit 15, is given to the remote side, i. e. the human-machine-interface 21 at the remote side 20. In particular, it is provided, that the medical instrument 10 can be selectively operated in a first mode giving the remote side 20 access to the medical instrument 10 and in a second mode giving the remote side 20 limited access to the medical instrument 10. For example, the remote side 20 can exchange both data and commands with the medical instrument 10, in particular with the control unit 15, in the first mode and can exchange only data in the second mode such as transferring image data sets corresponding to a current display of a screen of the medical instrument.

For getting access to the medical instrument 10 from the remote side, in particular the control unit 15, a request for accessing the medical instrument 10 is transmitted from the remote side 20 to the medical instrument 10. In particular, the medical instrument 10 is configured such that the medical instrument 10 is transferred into the first mode or into the second mode in response to the request by using a human-machine-interface 11 at the medical instrument 10. In other word: the first operators determines, whether the medical instrument 10 is operated in the first mode or the second mode. It is also thinkable that the access to the medical instrument 10 is refused by the human-machine-interface 11 of the medical instrument. Thus, it is advantageously possible to control the access by the human-machine interface 11 of the medical instrument. As a consequence, the first operator being located near to the medical instrument 10 can control the access of the second operator at the remote side 20 for safety reasons, provided that the first person is present at the medical instrument 1 or near to the human-machine-interface 11 of the medical instrument 10.

Furthermore, it is provided that the medical instrument 10 can be transferred selectively into the first mode by using the human-machine interface 21 at a remote side 20, when the human-machine-interface 11 of the medical instrument 10 is inactive for a defined period of time after requesting the access. Thus, it is advantageously possible to getting access to the medical instrument 10 at the remote side 20 even when the first operator is not present at the human-machine-interface 11 of the medical instrument 10, i. e. when the medical instrument 10 is unattended.

Furthermore, it is provided that the control unit 15 is configured such that the chance for transferring the medical instrument 10 in a first mode and/or the second mode is only given, when the medical instrument 10 is in a non-use state and the human-machine-interface 11 of the medical instrument 10 is inactive for a defined period of time after requesting the access. Thus, it can be avoided that settings are changed accidentally during a treatment of a patient. A non-use state might be identified by a login state of a patient, i. e. when no patient is logged in, the medical instrument 10 is in the non-use state.

Furthermore, the control unit 15 comprises a management system. Preferably, the management system is configured such that the management system defines the extent of access intended for the remote side. For example, a second operator of the first kind can choose between the first mode and the second mode and a second operator of the second kind can only select the second mode. Furthermore it also thinkable that several second modes are provided and the second mode is adapted individually to the second operator of the second kind. Basis for this is an assignment between the respective second operator and the individual defined second mode in the management system, in particular in a memory device of the management system. It is also thinkable that the management system is incorporated into a server, such as a network or a cloud.

## Claims

1. Method for using a medical instrument (10), in particular a medical imaging device, wherein the medical instrument (10) is selectively operable in a first mode giving access to the medical instrument (10) and in a second mode giving limited access, in particular refusing access, to the medical instrument (10), comprising the steps:
- requesting access to the medical instrument (10);
- transferring the medical instrument (10) into the first mode or into the second mode in response to the request by using a human-machine-interface (11) of the medical instrument (10) and
- transferring the medical instrument (10) into the first mode by using a human-machine interface (21) at a remote side (20), when the human-machine-interface (11) of the medical instrument (10) is inactive for a defined period of time, in particular after requesting the access.

2. Method according to claim 1, further comprising the step
- transferring the medical instrument (10) into the first mode, when the human-machine-interface (11) of the medical instrument (10) is inactive for a defined period of time after requesting the access and the medical instrument (10) is in a non-use state.

3. Method according to one of the preceding claims, further comprising
- transferring the medical instrument (10) into the first mode or into the second mode automatically, when the human-machine-interface (11) of the medical instrument (10) is inactive for a defined period of time before the request.

4. Method according to one of the preceding claims, wherein an information about a remaining time in the first mode or the second mode is provided at the remote side (20).

5. Method according to one of the preceding claims, wherein the first mode and/or the second mode are terminated by the human-machine-interface (11) at the medical instrument (10) or by the human-machine-interface (21) at the remote side (20).

6. Method according to one of the preceding claim, wherein transferring the medical instrument (10) into the first mode or into the second mode by using a human-machine interface (21) at a remote side (20) is selected by a management system.

7. Method according to one of the preceding claims, wherein the management system is incorporated into the medical instrument (10) or into a server.

8. Method according to one of the preceding claims, wherein a bidirectional communication between the medical instrument (10) and the remote side (20) is established in the first mode and a unidirectional communication is established in the second mode.

9. Method according to one of the preceding claims, wherein an information about the present usage of the medical instrument (10) is provided at the remote side (20).

10. Method according to one of the preceding claims, wherein in the first mode and/or the second mode administrative tasks are performed.

11. System comprising a medical instrument (10) and a remote side (20), wherein the system is configured for
- requesting access;
- transferring the medical instrument (10) into the first mode or into the second mode in response to the request by using a human-machine-interface (11) at the medical instrument (10) and
- transferring the medical instrument into the first mode by using a human-machine interface (11) at the remote side (20), when the human-machine-interface (11) pf the medical instrument is inactive, in particular for a defined period of time after requesting the access.

12. Computer program product for carrying out the steps of the method to any of the claims 1 to 10, when the computer program product is loaded into a memory of a programmable device.

13. A computer-readable medium on which is stored program elements than can be read and executed by a computer unit in order to perform steps of the method according to any of the claims 1 to 10 when the program elements are executed by the computer unit.

## Amended claims

### Amended claims in accordance with Rule 137(2) EPC.

1. Method for using a medical instrument (10), wherein the medical instrument is a medical imaging device, wherein the medical instrument (10) is selectively operable in a first mode giving access to the medical instrument (10) and in a second mode giving limited access, in particular refusing access, to the medical instrument (10), comprising the steps:
- requesting access to the medical instrument (10);
- transferring the medical instrument (10) into the first mode or into the second mode in response to the request by using a human-machine-interface (11) of the medical instrument (10) and
- transferring the medical instrument (10) into the first mode by using a human-machine interface (21) at a remote side (20), when the human-machine-interface (11) of the medical instrument (10) is inactive for a defined period of time, in particular after requesting the access, wherein transferring the medical instrument (10) into the first mode or the second mode by using a human-machine interface (21) at the remote side (20) is selected by a management system, wherein the management system defines the extend of access intended for the remote side, wherein the management system is incorporated into a server.

2. Method according to claim 1, further comprising the step
- transferring the medical instrument (10) into the first mode, when the human-machine-interface (11) of the medical instrument (10) is inactive for a defined period of time after requesting the access and the medical instrument (10) is in a non-use state.

3. Method according to one of the preceding claims, further comprising
- transferring the medical instrument (10) into the first mode or into the second mode automatically, when the human-machine-interface (11) of the medical instrument (10) is inactive for a defined period of time before the request.

4. Method according to one of the preceding claims, wherein an information about a remaining time in the first mode or the second mode is provided at the remote side (20).

5. Method according to one of the preceding claims, wherein the first mode and/or the second mode are terminated by the human-machine-interface (11) at the medical instrument (10) or by the human-machine-interface (21) at the remote side (20) .

6. Method according to one of the preceding claims, wherein a bidirectional communication between the medical instrument (10) and the remote side (20) is established in the first mode and a unidirectional communication is established in the second mode.

7. Method according to one of the preceding claims, wherein an information about the present usage of the medical instrument (10) is provided at the remote side (20).

8. Method according to one of the preceding claims, wherein in the first mode and/or the second mode administrative tasks are performed.

9. System comprising a medical instrument (10) and a remote side (20), wherein the system is configured for
- requesting access;
- transferring the medical instrument (10) into the first mode or into the second mode in response to the request by using a human-machine-interface (11) at the medical instrument (10) and
- transferring the medical instrument into the first mode by using a human-machine interface (11) at the remote side (20), when the human-machine-interface (11) pf the medical instrument is inactive, in particular for a defined period of time after requesting the access, wherein transferring the medical instrument (10) into the first mode or the second mode by using a human-machine interface (21) at the remote side (20) is selected by a management system, wherein the management system defines the extend of access intended for the remote side, wherein the management system is incorporated into a server.

10. Computer program product for carrying out the steps of the method to any of the claims 1 to 8, when the computer program product is loaded into a memory of a programmable device.

11. A computer-readable medium on which is stored program elements than can be read and executed by a computer unit in order to perform steps of the method according to any of the claims 1 to 8 when the program elements are executed by the computer unit.
